(19) Europäisches Patentamt
European Patent Office
Office européen des brevets

(11) EP 0 508 358 B1

(12) EUROPEAN PATENT SPECIFICATION

(45) Date of publication and mention of the grant of the patent:
**16.07.1997 Bulletin 1997/29**

(51) Int Cl.[6]: **C11D 3/386**, C11D 3/37

(21) Application number: **92105956.4**

(22) Date of filing: **07.04.1992**

(54) **Laundry detergent composition**

Waschmittel

Composition détergente pour le linge

(84) Designated Contracting States:
**DE ES FR GB IT NL**

(30) Priority: **12.04.1991 EP 91870062**

(43) Date of publication of application:
**14.10.1992 Bulletin 1992/42**

(73) Proprietor: **THE PROCTER & GAMBLE COMPANY**
**Cincinnati, Ohio 45202 (US)**

(72) Inventors:
- **Busch, Alfred**
**B-1840 Londerzeel (BE)**
- **MacCorquodale, Finlay**
**B-1200 Woluwe-St-Lambert (BE)**

(74) Representative: **Canonici, Jean-Jacques et al**
**Procter & Gamble European Technical Center N.V.**
**Temselaan 100**
**1853 Strombeek-Bever (BE)**

(56) References cited:
**EP-A- 0 350 098** **EP-A- 0 351 162**
**EP-A- 0 372 291** **EP-A- 0 508 034**
**WO-A-89/09259** **WO-A-91/17243**
**WO-A-91/19807** **GB-A- 2 094 826**
**GB-A- 2 137 221** **US-A- 4 435 307**

- **CHEMICAL ABSTRACTS, vol. 111, no. 26, 26 April 1989, Columbus, Ohio, US; abstract no. 235677, & JP-A-1132698 (LION)' page 150**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 508 358 B1

## Description

### Technical field

The present invention relates to the laundering of fabrics. Wash solutions are disclosed which are specifically designed for the washing of coloured fabrics. The wash solutions according to the present invention maintain the clarity of colours, especially upon repeated laundering.

### Background

Laundry detergent compositions are well known in the art and it is known to use vinylpyrrolidone polymers in such compositions, as described in EP 262 897 and EP 256 6969.

It is also known to use such polymers in detergent compositions specifically designed for the washing of colored fabrics, as in EP-A-265 257 which discloses compositions containing a vinylpyrrolidone polymer, a carboxylate polymer and a carboxy methyl cellulose, and EP 372 291 which describes detergent compositions comprising a specific surfactant mixture and a vinyl pyrrolidone polymer. More specifically, it is known that vinyl pyrrolidone polymers act as dye transfer inhibitors in the laundering process, as described in DE 28 14 287 and DE 28 14 329.

It is also known to use enzymes in laundry detengent compositions, including cellulase. Cellulase is known to have some effect on maintaining colours of fabrics inasmuch as cellulase controls the pilling of fabrics. This is described for instance in EP 177 165.

WO 89/09259 discloses a cellulase preparation useful for reducing the harshness of cotton-containing fabrics. Japanese patent 01, 132, 698 (Chem abstracts 111, 235677) discloses a high bulk density detergent composition containing cellulase. GB-A-2,094,826 discloses a cellulase enzyme detergent composition. EP-A-351,162 discloses a stabilised enzyme dispersion.

It has now been found that superior fabric colour maintenance could be achieved upon laundering by using a detergent composition comprising both a polyvinylpyrrolidone and cellulase; the combined effect of both these ingredients has surprisingly been found to be superior than the addition of the effects obtained by either alone.

### Summary of the invention

According to the invention there is provided a laundry wash solution comprising a solution of a laundry detergent composition comprising conventional detergency ingredients, characterized in that it comprises a cellulase, having a pH optimum of between 5 and 9,5 at a level of from 0.005 to 40 mg/l in said wash solution of said cellulase, and a polyvinylpyrrolidone of a molecular weight of from 8 000 to 15 000 at a level of from 5 to 500 mg/l of said polyvinylpyrrolidone in the wash solution.

### Detailed description of the invention.

The laundering detergent compositions for use in accord with the present invention comprise conventional detergency ingredients, including surfactants, builders and minor ingredients.

Suitable surfactants for use in the compositions according to the present invention include anionic surfactants such as water-soluble salts of alkyl benzene sulphonates, alkyl sulphates, alkyl polyethoxy ether sulphates, paraffin sulphonates, alpha-oleofin sulphonates, alpha-sulphoalkylcarboxylates and their esters, alkyl glyceryl ether sulphonates, fatty acid monoglyceride sulphates and sulphonates, alkyl phenol polyethoxyl ether sulphates, 2-acyloxy-alkane-1-sulphonates, and beta-alkyloxy sulphonates.

Especially preferred alkyl benzene sulphonates have 9 to 15 carbon atoms in a linear or branched alkyl chain, especially from 11 to 13 carbon atoms. Suitable alkyl sulphates have from 10 to 22 carbon atoms in the alkyl chain, more especially from 12 to 18 carbon atoms. Suitable alkyl polyethoxy ether sulphates have from 10 to 18 carbon atoms in the alkyl chain and have an average of from 1 to 23 - $CH_2CH_2O$-groups per molecule, especially from 10 to 16 carbon atoms in the alkyl chain and an average of from 1 to 6 -$CH_2$-$CH_2$-O groups per molecule.

Suitable paraffin sulphonates are essentially linear and contain from 8 to 24 atoms, more especially from 14 to 18 carbon atoms. Suitable alpha-oleofin sulphonates have from 10 to 24 carbon atoms, more especially from 14 to 16 carbon atoms; alpha-oleofin sulphonates can be made by reaction with sulphur trioxide, followed by neutralization under conditions such that any sultones present are hydrolyzed to the corresponding hydroxy alkane sulphonates. Suitable alpha-sulphocarboxylates contain from 6 to 20 carbon atoms; included herein are not only the salts of alpha-sulphonated fatty acids but also their esters made from alcohols containing 1 to 14 carbon atoms.

Suitable alkyl glycerly ether sulphates are ethers of alcohols having from 10 to 18 carbon atoms, more especially those derived from coconut oil and tallow. Suitable alkyl phenol polyethoxy ether sulphates have from 8 to 12 carbon

atoms in the alkyl chain and an average of from 1 to 6 -$CH_2CH_2O$- groups per molecule. Suitable 2-acyloxyalkane-1-sulphonates contain from 2 to 9 carbon atoms in the acyl group and from 9 to 23 carbon atoms in the alkane moiety. Suitable beta-alkyloxy alkane sulphonates contain from 1 to 3 carbon atoms in the alkyl group and from 8 to 20 carbon atoms in the alkane moiety.

Nonionic surfactants suitable for use in the compositions herein, are water-soluble ethoxylated materials of HLB 11.5-17.0 and include $c_{10}$-$_{20}$ primary and secondary alcohol ethoxylates and $C_6$-$_{10}$ alkylphenol ethoxylates. $C_{14-18}$ linear primary alcohols condensed with from seven to thirty moles of ethylene oxide per mole of alcohol are preferred examples being $C_{14}$-$C_{15}(EO)_7$, $C_{16-18}(E0)_{25}$ and especially $C_{16-18}(E0)_{11}$.

Another class of nonionic surfactants comprises alkyl polyglucoside compounds of general formula

$$RO\ (C_nH_{2n}0)_tZ_x$$

wherein Z is a moiety derived from glucose; R is an saturated hydrophobic alkyl group that contains from 12 to 18 carbon atoms; t is from 0 to 10 and n is 2 or 3; x is from 1.3 to 4, the compounds including less than 10% unreacted fatty alcohol and less than 50% short chain alkyl polyglucosides. Compounds of this type and their use in detergent are disclosed in EP-B 0 070 077, 0 075 996 and 0 094 118.

Also suitable as nonionic surfactants are poly hydroxy fatty acid amide surfactants of the formula

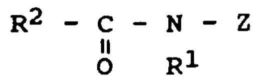

wherein $R^1$ is H, $C_{1-4}$ hydrocarbyl, 2-hydroxy ethyl, 2-hydroxy propyl or a mixture thereof, $R_2$ is $C_{5-31}$ hydrocarbyl, and Z is a polyhydroxyhydrocarbyl having a linear hydrocarbyl chain with at least 3 hydroxyls directly connected to the chain, or an alkoxylated derivative thereof. Preferably, $R^1$ is methyl, $R^2$ is a straight $C_{11-15}$ alkyl or alkenyl chain such as coconut alkyl or mixtures thereof, and Z is derived from a reducing sugar such as glucose, fructose, maltose, lactose, in a reductive amination reaction.

Other types of surfactants can be used, such as zwitteronic amphoteric, as well as cationic surfactants.

Cationic co-surfactants which can be used herein, include water-soluble quaternary ammonium compounds of the form $R_4R_5R_6R_7N^+X^-$, wherein $R_4$ is alkyl having from 10 to 20, preferably from 12-18 carbon atoms, and $R_5$, $R_6$ and $R_7$ are each $C_1$ to $C_7$ alkyl preferably methyl; $X^-$ is an anion, e.g. chloride. Examples of such trimethyl ammonium compounds include $C_{12-14}$ alkyl trimethyl ammonium chloride and cocalkyl trimethyl ammonium methosulfate. The compositions for use in accord with the present invention comprise from 1-70% by weight of surfactant, preferably from 10% to 30%, most preferably from 15%-25%.

<u>Builders</u>

Suitable builders for use herein include the nitrilotriacetates, polycarboxylates, citrates, water-soluble phosphates such as tri-polyphosphate and sodium ortho- and pyro-phosphates, and mixtures thereof. Metal ion sequestrants include all of the above, plus materials like ethylenediaminetetraacetate, the aminopolyphosphonates and a wide variety of other poly-functional organic acids and salts too numerous to mention in detail here. See U.S. Patent 3,579,454 for typical examples of the use of such materials in various cleaning compositions. Preferred polyfunctional organic acids species for use herein are citric acid, ethylene diamine tetramethylenephosphonic acid, and diethylene triaminepentamethylenephosphonic acid.

A further class of detergency builder materials useful in the present invention are insoluble sodium aluminosilicates. The 1-10 micrometer size zeolite (e.g., zeolite A) builder disclosed in German Patent 24 22 655 are especially preferred for use in low-phosphate compositions.

The compositions herein can also contain fatty acids, saturated or unsaturated, and the corresponding soaps. Suitable fatty acids, saturated or unsaturated, have from 10 to 18 carbon atoms in the alkyl chain. Preferred are unsaturated species having from 14 to 18 carbon atoms in the alkyl chain, most preferably oleic acid. The corresponding soaps can also be used.

The compositions herein can also contain compounds of the general formula $R\text{-}CH(COOH)CH_2(COOH)$ i.e. derivatives of succinic acid, wherein R is $C_{10\text{-}C20}$ alkyl or alkenyl, preferably $C_{12\text{-}C16}$ or wherein R may be substituted with hydroxyl, sulfo, sulfoxy or sulfone substituents.

The succinate builders are preferably used in the form of their water-soluble salts, including the sodium, potassium, ammonium and alkanolammonium salts.

Specific examples of succinate builders include : lauryl succinate, myristyl succinate, palmityl succinate, 2-dodecenyl succinate (preferred), 2-pentadecenyl succinate, and the like.

Also useful as builders in the present context are the compounds described in US Patent 4,663,071, i.e. mixtures of tartrate monosuccinic acid and tartrate disuccinic acid in weight ratio of monosuccinic to disuccinic of from 97:3 to 20:80, preferably 95:5 to 40:6.

Compositions for use in accord with the present invention comprise from 1% to 70% of a builder, preferably from 30% to 60%, most preferably from 40% to 50% .

The compositions for use in accord with the present invention are characterized in that they comprise an alkaline cellulase and a polyvinylpyrrolidone. It is this specific combination of ingredients which provides the superior fabric colour care properties of the wash solution according to the present invention. These fabric colour care benefits are better obtained when the fabrics are repeatedly washed with the wash solution according to the present invention - thus the present invention also compasses a method of washing fabrics wherein the fabrics are repeatedly washed with a wash solution according to the present invention.

## The cellulase

The cellulase usable in the present invention may be any bacterial or fungal cellulase, having a pH optimum of between 5 and 9.5.

Suitable cellulase are disclosed in GB-A-2 075 028 and GB-A-2 095 275.

Examples of such cellulases are cellulase produced by a strain of Humicola insolens (Humicola grisea var. thermoidea), particularly by the Humicola strain DSM 1800, and cellulases produced by a funges of Bacillus N or a cellulase 212-producing fungus belonging to the genus Aeromonas, and cellulase extracted from the hepatopancreas of a marine mullosc (Dolabella Auricula Solander).

The cellulase added to the composition of the invention may be in the form of a non-dusting granulate, e.g. "marumes" or "prills", or in the form of a liquid in which the cellulase is provided as a cellulase concentrate suspended in e.g. a nonionic surfactant or dissolved in an aqueous medium.

Preferred cellulases for use herein are characterized in that said cellulase they provide at least 10% removal of immobilized radioactive labelled carboxymethylcellulose according to the C14CMC-method at $25x10^{-6}$% by weight of cellulase protein in the laundry test solution.

Other suitable water-soluble organic salts are the homo- or co-polymeric acids or their salts, in which the polycarboxylic acid comprises at least two carboxyl radicals separated from each other by not more than two carbon atoms.

Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

## CELLULASE

The activity of enzymes and particularly the activity of cellulase enzyme has been defined for various applications by different analytical methods. These methods all attempt to provide a realistic assessment of the expected in use performance or at least a measurement correlating with the in use performance. As has been detailed in European Patent Application EP-A-350098, many of the methods, particularly these frequently used by cellulase manufacturers, are not sufficiently correlated with the in use performance of cellulase in laundry detergent compositions. This is due to the various other usage conditions for which these activity measurement methods have been developed.

The method described in EP-A-350098, has been developed to be and to have a predictive correlation for the ranking of cellulase activity in laundry detergent compositions.

The present invention therefore uses the method disclosed in EP-A-350098 to screen cellulases in order to distinguish preferred cellulases which are useful in the present invention. The screening method, hereinafter referred to as C14CMC-Method, which has been adopted from the method disclosed in EP-A-350098, can be described as follows :

### Principle :

The principle of the C14CMC-Method for screening is to measure at a defined cellulase concentration in a wash solution the removal of immobilized carboxy methyl cellulose (CMC) from a cloth substrate. The removal of CMC is measured by radio-active labelling of some of the CMC by using C14 radio-active carbon. Simple counting of the amount of radio-active C14 on the cloth substrate before and after the cellulase treatment allows the evaluation of the cellulase activity.

Sample preparation :

**CMC preparation :** The radio-active CMC stock solution is prepared according to Table I. The radio-active CMC can be obtained by methods referred to in EP-A-350098.

**Fabric substrates :** The fabric substrates are muslin cotton swatches having a size of 5 cm x 5 cm. They are inocculated with 0.35 ml of the radio-active labelled CMC stock solution in their center. The muslin cotton swatches are then airdried.

**Immobilization of CMC :** To immobilize the radio-active labelled CMC on the muslin cotton swatches, launderometer equipment " Linitest Original Haunau " made by Original Haunau, Germany, is used. A metal jar of the laundero-meter is filled with 400 ml of hard water (4 mmol/liter of $Ca^{++}$ ions). A maximum number of 13 swatches can be used per jar. The jar is then incubated in a heat-up cycle from 20°C to 60°C over 40 minutes in the laundero-meter equipment. After incubation the swatches are rinsed under running city water for 1 minute. They are squeezed and allowed to airdry for at least 30 minutes.

According to EP-A-350098 samples of the swatches with immobilized radio-active CMC can also be measured as "blank samples" without washing.

Sample treatment :

**Laundry test solution :** The laundry test solution is prepared according to the composition of Table II. It is balanced to pH 7.5. The laundry test solution is the basis to which a cellulase test sample is added. Care should be taken to not dilute the laundry test solution by adding water to a 100% balance prior to having determined the amount of cellulase to be added. The amount of cellulase which is used in this screening test should be added to provide 25 x $10^{-6}$ weight percent of cellulase protein in the laundry test solution (equivalent to 0.25 milligram/liter at 14.5 °C).

**Wash procedure :** The swatches thus inocculated with radio-active labelled CMC are then treated in a laundry simulation process. The laundry process is simulated in the laundero-meter type equipment," Linitest, Original Haunau", by Original Haunau, Haunau Germany. An individual swatch is put into a 20 $cm^3$ glass vial. The vial is filled with 10 ml of the laundry test solution and then sealed liquid tight. Up to 5 vials are put into each laundero-meter jar. The jar is filled with water as a heat tranfer medium for the laundering simulation. The laundering simulation is conducted as a heat-up cycle from 20°C to 60°C over 40 minutes.

After the processing of the samples the vials are submerged in cold water and subsequently each swatch is taken out of its vial, rinsed in a beaker under running soft water, squeezed and allowed to airdry for at least 30 minutes.

Measurement :

In order to measure radio-active labelled CMC removal, a scintillation counter, for example, a LKB 1210 Ultrabeta Scintillation Counter, is used. In order to obtain most accurate results, the instruction manual for optimum operation of the particular scintillation counter should be followed. For example, for the LKB 1210 Ultrabeta Scintillation Counter, the following procedure should be followed. The swatch to be measured is put into a plastic vial filled with 12 ml of scintillator liquid (e.g. scintillator 299 from Packard). The swatch is then allowed to stabilize for at least 30 minutes. The vial is then put into the LKB 1210 Ultrabeta Scintillation Counter and the respective radio-activity counts for the swatch is obtained.

In order to measure the amount of CMC removal due only to the cellulase, a measurement of a swatch which has been inocculated at the same time but has been treated in the laundry test solution without cellulase, is necessary. The activity of the cellulase is then expressed as percent of radio-active labelled CMC removal. This percentage is calculated by the following formula :

$$\text{\% of radio-active CMC removal} = \frac{XO - XC}{XO} \times 100$$

Wherein

XO is the radioactivity scintillation count of a swatch treated with the laundry test solution without cellulase
XC is the radioactivity scintillation count of a swatch treated with the laundry test solution containing the cellulase to be evaluated

**Statistical considerations, procedure confirmation :**

In order to provide statistically sound results, standard statistical analysis should be employed. For the given example, using the LKB 1210 Ultrabeta Scintillation Counter, it has been found that a sample size of 3 swatches for each radioactivity scintillation count can be used.

In order to confirm the procedure by internal crosschecking, measurement and calculation of the "blank sample" according to EP-A-350098 are recommended. This will allow to detect and eliminate errors.

Interpretation of results :

The described screening test does provide a fast, unique and reliable method to identify preferred cellulases which satisfy the activity criteria of the present invention.

It has been found that a removal of 10% or more of the immobilized radioactive labelled CMC according to the above C14CMC-method, indicates that the respective preferred cellulase satisfies the requirements of the invention.

It will be obvious to those skilled in the art that removal percentages above 10% indicate a higher activity for the respective cellulase. It therefore is contemplated that cellulase providing above 25% or preferably above 50% removal of radioactive labelled CMC, at the protein concentration in the laundry test solution according to the C14CMC-method, would provide indication of an even better performance of the cellulase for use in laundry detergents.

It also has been contemplated that usage of higher concentrations of cellulase for C14CMC-method, would provide higher removal percentages. However, there exists no linear proven correlation between cellulase concentration and removal percentage obtained by it.

It also has been contemplated that usage of higher concentrations of cellulase for C14CMC-method, would provide higher removal percentages.

TABLE I:

| Radioactive $C_{14}$ labelled CMC stock solution (all percentages by weight of total solution) | |
|---|---|
| Total CMC* (CMC should be detergent grade CMC with a degree of substitution from about 0.47 to about 0.7) | $99.2 \times 10^{-3}$% |
| Ethanol | $14985.12 \times 10^{-3}$% |
| Deionized Water | $84915.68 \times 10^{-3}$% |
| Total : | 100% |

* Total CMC contains non-radio-active and radio-active CMC to provide a radio-activity which allows sufficiently clear readings on the scintillation counter used. For example, the radio-active CMC can have an activity of 0.7 millicurie/g and be mixed

TABLE II:

| Laundry test solution (all percentages by weight of total solution) | |
|---|---|
| Linear $C_{12}$ alkyl benzene sulphonic acid | 0.110% |
| Coconut alkyl sulphate (TEA salt) | 0.040% |
| $C_{12-15}$ alcohol ethoxylate (E07) | 0.100% |
| Coconut fatty acid | 0.100% |
| Oleic acid | 0.050% |
| Citric acid | 0.010% |
| Triethanolamine | 0.040% |
| Ethanol | 0.060% |
| Propanediol | 0.015% |
| Sodium hydroxide | 0.030% |
| Sodium formate | 0.010% |
| Protease | 0.006% |
| Water (2.5 mmol/liter $Ca^{++}$), pH adjustment agent (HCL or NaOH solutions) and cellulase | balance to 100% |

According to the present invention, preferred cellulases are those as described in International Patent Application WO 91/17243. For example, a cellulase preparation useful in the compositions of the invention can consist essentially

of a homogeneous endoglucanase component, which is immunoreactive with an antibody raised against a highly purified 43kD cellulase derived from Humicola insolens, DSM 1800, or which is homologous to said 43kD endoglucanase.

It should be stressed that all preferred cellulase enzymes according to the present invention have to meet the criteria of the above mentioned screening test. However, in the Danish Patent Application 1159/90 additional criteria are established allowing to identify preferred cellulase enzymes in combination with the present screening test.

Cellulase preparations particularly useful in the compositions of the invention are those in which in addition to the screening test, the endoglucanase component exhibits a CMC-endoase activity of at least about 50, preferably at least about 60, in particular at least about 90 CMC-endoase units per mg of total protein. In particular, a preferred endoglucanase component exhibits a CMC-endoase activity of at least 100 CMC-endoase units per mg of total protein.

In the present context, the term "CMC-endoase activity" refers to the endoglucanase activity of the endoglucanase component in terms of its ability to degrade cellulose to glucose, cellobiose and triose, as determined by a viscosity decrease of a solution of carboxymethyl cellulose (CMC) after incubation with the cellulase preparation of the invention, as described in detail below.

The CMC-endoase (endoglucanase) activity can be determined from the viscosity decrease of CMC, as follows : A substrate solution is prepared, containing 35 g/l CMC (Hercules® 7 LFD) in 0.1 M tris buffer at pH 9.0. The enzyme sample to be analyzed is dissolved in the same buffer. 10 ml substrate solution and 0.5 ml enzyme solution are mixed and transferred to a viscosimeter (e.g. Haake VT 181, NV sensor, 181 rpm), thermostated at 40°C. Viscosity readings are taken as soon as possible after mixing and again 30 minutes later. The amount of enzyme that reduces the viscosity to one half under these conditions is defined as 1 unit of CMC-endoase activity.

SDS polyacrylamide gel electrophoresis (SDS-PAGE) and isoelectric focusing with marker proteins in a manner known to persons skilled in the art were used to determine the molecular weight and isolelectric point (pI), respectively, of the endoglucanase component in the cellulase preparation useful in the present context. In this way, the molecular weight of a specific endoglucanase component was determined to be 43kD. The isoelectric point of this endoglucanase was determined to be about 5.1.

The cellobiohydrolase activity may be defined as the activity towards cellobiose p-nitrophenyl. The activity is determined as µmole nitrophenyl released per minute at 37°C and pH 7.0. The present endoglucanase component was found to have essentially no cellobiohydrolase activity.

The endoglucanase component in the cellulase preparation herein has initially been isolated by extensive purification procedures, i.a. involving reverse phase HPLC purification of a crude H. insolens cellulase mixture according to U.S. 4,435,307. This procedure has surprisingly resulted in the isolation of a 43kD endoglucanase as a single component with unexpectedly favourable properties due to a surprisingly high endoglucanase activity.

Also, in addition to the screening test, the cellulase enzymes useful in the present compositions can further be defined as enzymes exhibiting endoglucanase activity (in the following referred to as an "endoglucanase enzyme"), which enzymes have the amino acid sequence shown in the appended Sequence Listing ID#2, or a homologue thereof exhibiting endoglucanase activity.

In the present context, the term "homologue" is intended to indicate a polypeptide encoded by DNA which hybridizes to the same probe as the DNA coding for the endoglucanase enzyme with this amino acid sequence under certain specified conditions (such as presoaking in 5xSSC and prehybridizing for 1 h at 40°C in a solution of 20% formamide, 5xDenhardt's solution, 50 mM sodium phosphate, pH 6.8, and 50 ug of denatured sonicated calf thymus DNA, followed by hybridization in the same solution supplemented with 100 uM ATP for 18 h at 40°C). The term is intended to include derivatives of the aforementioned sequence obtained by addition of one or more amino acid residues to either or both the C- and N-terminal of the native sequence, substitution of one or more amino acid residues at one or more sites in the native sequence, deletion of one or more amino acid residues at either or both ends of the native amino acid sequence or at one or more sites within the native sequence, or insertion of one or more amino acid residues at one or more sites in the native sequence.

The endoglucanase enzyme herein may be one producible by species of Humicola such as Humicola insolens e. g. strain DSM 1800, deposited on October 1, 1981 at the Deutsche Sammlung von Mikroorganismen, Mascheroder Weg 1B, D-3300 Braunschweig, FRG, in accordance with the provisions of the Budapest Treaty on the International Recognition of the Deposit of Microorganisms for the Purposes of Patent Procedure (the Budapest Treaty).

In still a further aspect, the cellulase enzymes useful herein can be defined, in addition to the screening test, as endoglucanase enzymes which have the amino acid sequence shown in the appended Sequence Listing ID#4, or a homologue thereof (as defined above) exhibiting endoglucanase activity. Said endoglucanase enzyme may be one producible by a species of Fusarium, such as Fusarium oxysporum, e.g. strain DSM 2672, deposited on June 6, 1983 at the Deutsche Sammlung von Mikroorganismen, Mascheroder Weg 1B, D-3300 Braunschweig, FRG, in accordance with the provisions of the Budapest Treaty.

Furthermore, it is contemplated that homologous endoglucanases may be derived from other microorganisms producing cellulolytic enzymes, e.g. species of Trichoderma, Myceliophthora, Phanerochaete, Schizophyllum, Penicillium, Aspergillus, and Geotricum.

For industrial production of the cellulase preparation herein, however, it is preferred to employ recombinant DNA techniques or other techniques involving adjustements of fermentations or mutation of the microorganisms involved to ensure overproduction of the desired enzymatic activities. Such methods and techniques are known in the art and may readily be carried out by persons skilled in the art.

The endoglucanase component may thus be one which is producible by a method comprising cultivating a host cell transformed with a recombinant DNA vector which carries a DNA sequence encoding said endoglucanase component or a precursor of said endoglucanase component as well as DNA sequences encoding functions permitting the expression of the DNA sequence encoding the endoglucanase component or precursor thereof, in a culture medium under conditions permitting the expression of the endoglucanase component or precursor thereof and recovering the endoglucanase component from the culture.

DNA constructs comprising a DNA sequence encoding an endoglucanase enzyme as described above, or a precursor form of the enzyme, include the DNA constructs having a DNA sequence as shown in the appended Sequence Listings ID#1 or ID#3, or a modification thereof. Examples of suitable mofidications of the DNA sequence are nucleotide substitutions which do not give rise to another amino acid sequence of the endoglucanase, but which correspond to the codon usage of the host organism into which the DNA construct is introduced or nucleotide substitutions which do give rise to a different amino acid sequence and therefore, possibly, a different protein structure which might give rise to an endoglucanase mutant with different properties than the native enzyme. Other examples of possible modifications are insertion of one or more nucleotides at either end of the sequence, or deletion of one or more nucleotides at either end or within the sequence.

DNA constructs encoding endoglucanase enzymes useful herein may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, annealed, ligated and cloned in suitable vectors.

A DNA construct encoding the endoglucanase enzyme or a precursor thereof may, for instance, be isolated by establishing a cDNA or genomic library of a cellulase-producing microorganism, such as Humicola insolens, DSM 1800, and screening for positive clones by conventional procedures such as by hybridization using oligonucleotide probes synthesized on the basis of the full or partial amino acid sequence of the endoglucanase in accordance with standard techniques (cf. Sambrook et al., Molecular Cloning: A Laboratory Manual, 2nd. Ed. Cold Spring Harbor, 1989), or by selecting for clones expressing the appropriate enzyme activity (i.e. CMC-endoase activity as defined above), or by selecting for clones producing a protein which is reactive with an antibody against a native cellulase (endoglucanase).

Finally, the DNA construct may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA construct, in accordance with standard techniques. The DNA construct may also be prepared by polymerase chain reaction using specific primers, for instance as described in US 4,683,202 or R.K. Saiki et al., Science 239, 1988, pp. 487-491.

Recombinant expression vectors into which the above DNA constructs are inserted include any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into wich it has been integrated.

In the vector, the DNA sequence encoding the endoglucanase should be operably connected to a suitable promoter and terminator sequence. The promoter may be any DNA sequence which shows transcriptional activity in the host cell of choice and may be derived from genes encoding proteins either homologous or heterologous to the host cell. The procedures used to ligate the DNA sequences coding for the endoglucanase, the promoter and the terminator, respectively, and to insert them into suitable vectors are well known to persons skilled in the art (cf., for instance, Sambrook et al., op.cit.).

Host cells which are transformed with the above DNA constructs or the above expression vectors may be for instance belong to a species of Aspergillus, most preferably Aspergillys oryzae or Aspergillus niger. Fungal cells may be transformed by a process involving protoplast formation and transformation of the protoplasts followed by regeneration of the cell wall in a manner known per se. The use of Aspergillus as a host microorganism is described in EP 238 023 (of Novo Industri A/S), the contents of which are hereby incorporated by reference. The host cell may also be a yeast cell, e.g. a strain of Saccharomyces cerevisiae.

Alternatively, the host organism may be a bacterium, in particular strains of Streptomyces and Bacillus, and E. coli. The transformation of bacterial cells may be performed according to conventional methods, e.g. as described in Sambrook et al., Molecular Cloning: A Laboratory Manual, Cold Spring Harbor, 1989.

The screening of appropriate DNA sequences and construction of vectors may also be carried out by standard procedures, cf. Sambrook et al., op.cit.

The medium used to cultivate the transformed host cells may be any conventional medium suitable for growing the host cells in question. The expressed endoglucanase may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

By employing recombinant DNA techniques as indicated above, techniques of protein purification, techniques of fermentation and mutation or other techniques which are well known in the art, it is possible to provide endoglucanases of a high purity.

The level in the wash solution of cellulase described above should be such that the amount of enzyme protein in the wash solution is from 0.005 to 40 mg/liter of wash solution, preferably 0.01 to 10 mg/liter of wash solution.

## The vinylpyrrolidone polymer

The wash solution according to the invention also comprises a polyvinylpyrrolidone having a molecular weight of from 8 000 to 15 000. The level in the wash solution according to the present invention of polyvinylpyrrolidone should be such that the amount of polyvinylpyrrolidone in the wash solution is from 5 to 500 mg/l, preferably from 15 to 100 mg/l, most preferably 25 mg/l to 75 mg/l.

## Optional ingredients

The compositions will typically include optional ingredients that normally form part of detergent compositions. Antiredeposition and soil suspension agents, optical brighteners, clays, bleaches, bleach activators, suds supressors, anticacking agents, dyes, perfumes and pigments are examples of such optional ingredients and can be added in varying amounts as desired.

The compositions can be in liquid, pasty or granular form, preferably granular. Granular compositions can also be in "compact form", i.e. they may have a relatively higher density than conventional granular detergents, i.e. from 550 to 950 g/l; in such case, the granular detergent compositions will contain a lower amount of "inorganic filler salt", compared to conventional granular detergents; typical filler salts are alkaline ether metal salts of sulphates and chlorides, typically sodium sulphate; "compact" detergents typically comprise not more than 10% filler salt. Liquid and conventional granular detergents are typically used at a concentration of from 1 to 2% by weight in the wash liquor, preferably 1.5%, whereas compact granules are used at a concentration of from 0.5% to 1.5% by weight, preferably 1%.

The following examples illustrate the present invention and the unexpected superior colour care benefits obtained therefrom.

## EXAMPLE I

The following composition was made:

| | |
|---|---|
| Linear alkyl benzene sulphonate (LAS) | 11% |
| Alkyl sulphate | 5% |
| Nonionic | 6% |
| Trisodium citrate | 15% |
| Zeolite | 32% |
| Polymer | 4% |
| Chelant | 0.2% |
| Sodium sulphate | 5% |
| Sodium silicate | 2% |

A load consisting of two types of coloured fabric (blue knit 91% cotton/9% polyester, and a peach coloured 100% cotton flannel material) was washed 10 times with this composition at a concentration in the wash liquor of 0.7%. In the 10th cycle an additional fabric was introduced which bled dye into the wash. In one experiment this was a red cotton fabric, in another this was a brown cotton fabric. Colour appearance on the washed load was then evaluated using a panel score with the following scale :

1. I think I see a difference with the reference
2. I definitely see a difference with the reference
3. I see a big difference with the reference
4. A day and night difference with the reference

The above detergent composition was then supplemented either with 1% PVP, or with 0.025% cellulase, or with both 0.025% cellulase and 1% PVP. Colour appearance was again evaluated as above. Results are summarized in the table below. A positive panel score indicates a better result (less colour transfer).

These results show that the action of PVP and cellulase together is unexpectedly superior than the sum of the individual actions of both these ingredients.

OVERALL COLOUR APPEARANCE BENEFITS - PVP + CELLULASE - PANEL SCORE UNITS

| | | Blue Knit (91% Cotton, 9% Polyester) | | Peach Flannel (100% Cotton) | |
|---|---|---|---|---|---|
| | | Red Dye Transfer | Brown Dye Transfer | Red Dye Transfer | Brown Dye Transfer |
| Reference - Aged Fabric at Start | No PVP, No Cellulase | -2.2 | -4.0 | -2.5 | -4.0 |
| | With Cellulase, No PVP | 1.8 | -3.5 | 2.5 | -4.0 |
| | With PVP, No Cellulase | -1.2 | -2.0 | -0.2 | -0.8 |
| | With PVP, With Cellulase | 1.5 | 1.0 | 3.5 | 1.5 |
| Reference - New Fabric at Start | No PVP, No Cellulase | -3.2 | -4.0 | -2.2 | -4.0 |
| | With Cellulase, No PVP | -1.5 | -4.0 | -1.8 | -4.0 |
| | With PVP, No Cellulase | -2.8 | -3.0 | -1.5 | -3.0 |
| | With PVP, With Cellulase | -1.2 | -1.5 | -0.5 | 0.0 |
| Reference - Aged Fabric Washed w/o PVP and w/o Cellulase | With Cellulase, No PVP | 2.8 | 1.8 | 1.8 | 0.2 |
| | With PVP, No Cellulase | 0.5 | 3.5 | 1.0 | 3.2 |
| | With PVP, With Cellulase | 4.0 | 3.8 | 4.0 | 4.0 |

EXAMPLES II to VII

The following compositions are made.

a) Compact granular detergent : examples II and III

| EXAMPLES | II | III |
|---|---|---|
| Linear alkyl benzene sulphonate | 11.40 | 10.70 |
| Tallow alkyl sulphate | 1.80 | 2.40 |
| $C_{45}$ alkyl sulphate | 3.00 | 3.10 |
| $C_{45}$ alcohol 7 times ethoxylated | 4.00 | 4.00 |
| Tallow alcohol 11 times ethoxylated | 1.80 | 1.80 |
| Dispersant | 0.07 | 0.1 |
| Silicone fluid | 0.80 | 0.80 |
| Trisodium citrate | 14.00 | 15.00 |
| Citric acid | 3.00 | 2.50 |
| Zeolite | 32.50 | 32.10 |
| Maleic acid acrylic acid copolymer | 5.00 | 5.00 |
| DETMPA | 1.00 | 0.20 |
| Cellulase (active protein) | 0.03 | 0.025 |
| Alkalase/BAN | 0.60 | 0.60 |
| Lipase | 0.36 | 0.40 |
| Sodium silicate | 2.00 | 2.50 |
| Sodium sulphate | 3.50 | 5.20 |
| PVP | 0.30 | 0.50 |

b) conventional granular detergent : examples IV and V

| EXAMPLES | IV | V |
|---|---|---|
| Sodium linear $C_{12}$ alkyl benzene sulfonate | 6.5 | 8.0 |
| Sodium sulfate | 15.0 | 18.0 |
| Zeolite A | 26.0 | 22.0 |
| Sodium nitrilotriacetate | 5.0 | 5.0 |
| Cellulase (active protein) | 0.02 | 0.03 |
| PVP | 0.5 | 0.7 |
| TAED | 3.0 | 3.0 |
| Boric acid | 4.0 | - |
| Minors | __ up to 100 __ | |

c) liquid detergent : examples VI and VII

| EXAMPLES | VI | VII |
|---|---|---|
| $C_{12-14}$ alkenyl succinic acid | 3.0 | 8.0 |
| Citric acid monohydrate | 10.0 | 15.0 |
| Sodium $C_{12-15}$ alkyl sulphate | 8.0 | 8.0 |
| Sodium sulfate of $C_{12-15}$ alcohol 2 times ethoxylated | - | 3.0 |
| $C_{12-15}$ alcohol 7 times ethoxylated | - | 8.0 |
| $C_{12-15}$ alcohol 5 times ethoxylated | 8.0 | - |
| Diethylene triamine penta (methylene phosphonic acid) | 0.2 | - |
| Oleic acid | 1.8 | - |
| Ethanol | 4.0 | 4.0 |

(continued)

| EXAMPLES | VI | VII |
|---|---|---|
| Propanediol | 2.0 | 2.0 |
| Protease | 0.2 | 0.2 |
| Cellulase (active protein) | 0.2 | 0.05 |
| PVP | 1.0 | 2.0 |
| Suds suppressor | 0.15 | 0.15 |
| NaOH | up to pH 7.5 | |
| Waters and minors | up to 100 parts | |

**INFORMATION FOR SEQ ID NO 1 :**

(i) SEQUENCE CHARACTERISTICS

(A)Length : 1060 base pairs
(B)Type : nucleic acid
(C)Strandedness : single
(D)Topology : linear

(ii) MOLECULE TYPE : cDNA

(iii) HYPOTHETICAL : NO

(iv) ORIGINAL SOURCE

(A) Organism : Humicola insolens
(B) Strain : DSM 1800

(ix) FEATURE

(A) Name/Key : mat_peptide
(B) Location : 73..927

(ix) FEATURE

(A) Name/Key : sig_peptide
(B) Location : 10..72

(ix) FEATURE

(A) Name/Key : CDS
(B) Location : 10..927

SEQUENCE DESCRIPTION: SEQ ID NO:1:

```
GGATCCAAG ATG CGT TCC TCC CCC CTC CTC CCG TCC GCC GTT GTG GCC          48
          Met Arg Ser Ser Pro Leu Leu Pro Ser Ala Val Val Ala
          -21 -20                 -15                 -10

GCC CTG CCG GTG TTG GCC CTT GCC GCT GAT GGC AGG TCC ACC CGC TAC        96
Ala Leu Pro Val Leu Ala Leu Ala Ala Asp Gly Arg Ser Thr Arg Tyr
            -5                   1               5

TGG GAC TGC TGC AAG CCT TCG TGC GGC TGG GCC AAG AAG GCT CCC GTG       144
Trp Asp Cys Cys Lys Pro Ser Cys Gly Trp Ala Lys Lys Ala Pro Val
    10                  15                  20

AAC CAG CCT GTC TTT TCC TGC AAC GCC AAC TTC CAG CGT ATC ACG GAC       192
Asn Gln Pro Val Phe Ser Cys Asn Ala Asn Phe Gln Arg Ile Thr Asp
25                  30                  35                  40

TTC GAC GCC AAG TCC GGC TGC GAG CCG GGC GGT GTC GCC TAC TCG TGC       240
Phe Asp Ala Lys Ser Gly Cys Glu Pro Gly Gly Val Ala Tyr Ser Cys
                45                  50                  55

GCC GAC CAG ACC CCA TGG GCT GTG AAC GAC GAC TTC GCG CTC GGT TTT       288
Ala Asp Gln Thr Pro Trp Ala Val Asn Asp Asp Phe Ala Leu Gly Phe
            60                  65                  70
```

p

```
GCT GCC ACC TCT ATT GCC GGC AGC AAT GAG GCG GGC TGG TGC TGC GCC        336
Ala Ala Thr Ser Ile Ala Gly Ser Asn Glu Ala Gly Trp Cys Cys Ala
        75                  80                  85

TGC TAC GAG CTC ACC TTC ACA TCC GGT CCT GTT GCT GGC AAG AAG ATG        384
Cys Tyr Glu Leu Thr Phe Thr Ser Gly Pro Val Ala Gly Lys Lys Met
    90                  95                  100

GTC GTC CAG TCC ACC AGC ACT GGC GGT GAT CTT GGC AGC AAC CAC TTC        432
Val Val Gln Ser Thr Ser Thr Gly Gly Asp Leu Gly Ser Asn His Phe
105                 110                 115                 120

GAT CTC AAC ATC CCC GGC GGC GGC GTC GGC ATC TTC GAC GGA TGC ACT        480
Asp Leu Asn Ile Pro Gly Gly Gly Val Gly Ile Phe Asp Gly Cys Thr
                125                 130                 135

CCC CAG TTC GGC GGT CTG CCC GGC CAG CGC TAC GGC GGC ATC TCG TCC        528
Pro Gln Phe Gly Gly Leu Pro Gly Gln Arg Tyr Gly Gly Ile Ser Ser
        140                 145                 150

CGC AAC GAG TGC GAT CGG TTC CCC GAC GCC CTC AAG CCC GGC TGC TAC        576
Arg Asn Glu Cys Asp Arg Phe Pro Asp Ala Leu Lys Pro Gly Cys Tyr
        155                 160                 165

TGG CGC TTC GAC TGG TTC AAG AAC GCC GAC AAT CCG AGC TTC AGC TTC        624
Trp Arg Phe Asp Trp Phe Lys Asn Ala Asp Asn Pro Ser Phe Ser Phe
    170                 175                 180

CGT CAG GTC CAG TGC CCA GCC GAG CTC GTC GCT CGC ACC GGA TGC CGC        672
Arg Gln Val Gln Cys Pro Ala Glu Leu Val Ala Arg Thr Gly Cys Arg
185                 190                 195                 200

CGC AAC GAC GAC GGC AAC TTC CCT GCC GTC CAG ATC CCC TCC AGC AGC        720
Arg Asn Asp Asp Gly Asn Phe Pro Ala Val Gln Ile Pro Ser Ser Ser
                205                 210                 215

ACC AGC TCT CCG GTC AAC CAG CCT ACC AGC ACC AGC ACC ACG TCC ACC        768
Thr Ser Ser Pro Val Asn Gln Pro Thr Ser Thr Ser Thr Thr Ser Thr
            220                 225                 230

TCC ACC ACC TCG AGC CCG CCA GTC CAG CCT ACG ACT CCC AGC GGC TGC        816
Ser Thr Thr Ser Ser Pro Pro Val Gln Pro Thr Thr Pro Ser Gly Cys
        235                 240                 245

ACT GCT GAG AGG TGG GCT CAG TGC GGC GGC AAT GGC TGG AGC GGC TGC        864
Thr Ala Glu Arg Trp Ala Gln Cys Gly Gly Asn Gly Trp Ser Gly Cys
    250                 255                 260

ACC ACC TGC GTC GCT GGC AGC ACT TGC ACG AAG ATT AAT GAC TGG TAC        912
Thr Thr Cys Val Ala Gly Ser Thr Cys Thr Lys Ile Asn Asp Trp Tyr
265                 270                 275                 280

CAT CAG TGC CTG TAGACGCAGG GCAGCTTGAG GGCCTTACTG GTGGCCGCAA            964
His Gln Cys Leu
                285

CGAAATGACA CTCCCAATCA CTGTATTAGT TCTTGTACAT AATTTCGTCA TCCCTCCAGG     1024

GATTGTCACA TAAATGCAAT GAGGAACAAT GAGTAC                               1060
```

INFORMATION FOR SEQ ID NO 2 :

(i) SEQUENCE CHARACTERISTICS

(A) Length : 305 amino acids
(B) Type : amino acid
(D) Topology : linear

(ii) MOLECULE TYPE : protein

SEQUENCE DESCRIPTION: SEQ ID NO:2:

```
Met Arg Ser Ser Pro Leu Leu Pro Ser Ala Val Val Ala Ala Leu Pro
-21 -20                 -15                     -10

Val Leu Ala Leu Ala Ala Asp Gly Arg Ser Thr Arg Tyr Trp Asp Cys
 -5                  1               5                   10

Cys Lys Pro Ser Cys Gly Trp Ala Lys Lys Ala Pro Val Asn Gln Pro
            15                  20                  25

Val Phe Ser Cys Asn Ala Asn Phe Gln Arg Ile Thr Asp Phe Asp Ala
        30                  35                  40

Lys Ser Gly Cys Glu Pro Gly Gly Val Ala Tyr Ser Cys Ala Asp Gln
    45                  50                  55

Thr Pro Trp Ala Val Asn Asp Asp Phe Ala Leu Gly Phe Ala Ala Thr
60                  65                  70                  75

Ser Ile Ala Gly Ser Asn Glu Ala Gly Trp Cys Cys Ala Cys Tyr Glu
                80                  85                  90

Leu Thr Phe Thr Ser Gly Pro Val Ala Gly Lys Lys Met Val Val Gln
            95                  100                 105

Ser Thr Ser Thr Gly Gly Asp Leu Gly Ser Asn His Phe Asp Leu Asn
        110                 115                 120

Ile Pro Gly Gly Gly Val Gly Ile Phe Asp Gly Cys Thr Pro Gln Phe
    125                 130                 135

Gly Gly Leu Pro Gly Gln Arg Tyr Gly Gly Ile Ser Ser Arg Asn Glu
140                 145                 150                 155

Cys Asp Arg Phe Pro Asp Ala Leu Lys Pro Gly Cys Tyr Trp Arg Phe
                160                 165                 170

Asp Trp Phe Lys Asn Ala Asp Asn Pro Ser Phe Ser Phe Arg Gln Val
            175                 180                 185

Gln Cys Pro Ala Glu Leu Val Ala Arg Thr Gly Cys Arg Arg Asn Asp
        190                 195                 200

Asp Gly Asn Phe Pro Ala Val Gln Ile Pro Ser Ser Ser Thr Ser Ser
    205                 210                 215
```

```
Pro Val Asn Gln Pro Thr Ser Thr Ser Thr Thr Ser Thr Ser Thr Thr
220                 225                 230                 235

Ser Ser Pro Pro Val Gln Pro Thr Thr Pro Ser Gly Cys Thr Ala Glu
                240                 245                 250

Arg Trp Ala Gln Cys Gly Gly Asn Gly Trp Ser Gly Cys Thr Thr Cys
            255                 260                 265

Val Ala Gly Ser Thr Cys Thr Lys Ile Asn Asp Trp Tyr His Gln Cys
        270                 275                 280

Leu
```

INFORMATION FOR SEQ ID NO 3

(i) SEQUENCE CHARACTERISTICS

(A) LENGTH : 1473 base pairs
(B) TYPE : nucleic acid
(C) STRANDEDNESS : single
(D) TOPOLOGY : linear

(ii) MOLECULE TYPE : cDNA

(iii) HYPOTHETICAL : NO

(iv) ANTI-SENSE : NO

(vi) ORIGINAL SOURCE

(A) ORGANISM : fusarium oxysporum
(B) STRAIN : DSM 2672

(ix) FEATURE

(A) NAME/KEY : CDS
(B) LOCATION : 97..1224

SEQUENCE DESCRIPTION: SEQ ID NO:3:

```
GAATTCGCGG CCGCTCATTC ACTTCATTCA TTCTTTAGAA TTACATACAC TCTCTTTCAA      60

AACAGTCACT CTTTAAACAA AACAACTTTT GCAACA ATG CGA TCT TAC ACT CTT       114
                                        Met Arg Ser Tyr Thr Leu
                                          1               5

CTC GCC CTG GCC GGC CCT CTC GCC GTG AGT GCT GCT TCT GGA AGC GGT       162
Leu Ala Leu Ala Gly Pro Leu Ala Val Ser Ala Ala Ser Gly Ser Gly
            10                  15                  20

CAC TCT ACT CGA TAC TGG GAT TGC TGC AAG CCT TCT TGC TCT TGG AGC       210
His Ser Thr Arg Tyr Trp Asp Cys Cys Lys Pro Ser Cys Ser Trp Ser
        25                  30                  35

GGA AAG GCT GCT GTC AAC GCC CCT GCT TTA ACT TGT GAT AAG AAC GAC       258
Gly Lys Ala Ala Val Asn Ala Pro Ala Leu Thr Cys Asp Lys Asn Asp
    40                  45                  50
```

AAC CCC ATT TCC AAC ACC AAT GCT GTC AAC GGT TGT GAG GGT GGT GGT 306
Asn Pro Ile Ser Asn Thr Asn Ala Val Asn Gly Cys Glu Gly Gly Gly
55 60 65 70

TCT GCT TAT GCT TGC ACC AAC TAC TCT CCC TGG GCT GTC AAC GAT GAG 354
Ser Ala Tyr Ala Cys Thr Asn Tyr Ser Pro Trp Ala Val Asn Asp Glu
75 80 85

CTT GCC TAC GGT TTC GCT GCT ACC AAG ATC TCC GGT GGC TCC GAG GCC 402
Leu Ala Tyr Gly Phe Ala Ala Thr Lys Ile Ser Gly Gly Ser Glu Ala
90 95 100

AGC TGG TGC TGT GCT TGC TAT GCT TTG ACC TTC ACC ACT GGC CCC GTC 450
Ser Trp Cys Cys Ala Cys Tyr Ala Leu Thr Phe Thr Thr Gly Pro Val
105 110 115

AAG GGC AAG AAG ATG ATC GTC CAG TCC ACC AAC ACT GGA GGT GAT CTC 498
Lys Gly Lys Lys Met Ile Val Gln Ser Thr Asn Thr Gly Gly Asp Leu
120 125 130

GGC GAC AAC CAC TTC GAT CTC ATG ATG CCC GGC GGT GGT GTC GGT ATC 546
Gly Asp Asn His Phe Asp Leu Met Met Pro Gly Gly Gly Val Gly Ile
135 140 145 150

TTC GAC GGC TGC ACC TCT GAG TTC GGC AAG GCT CTC GGC GGT GCC CAG 594
Phe Asp Gly Cys Thr Ser Glu Phe Gly Lys Ala Leu Gly Gly Ala Gln
155 160 165

TAC GGC GGT ATC TCC TCC CGA AGC GAA TGT GAT AGC TAC CCC GAG CTT 642
Tyr Gly Gly Ile Ser Ser Arg Ser Glu Cys Asp Ser Tyr Pro Glu Leu
170 175 180

CTC AAG GAC GGT TGC CAC TGG CGA TTC GAC TGG TTC GAG AAC GCC GAC 690
Leu Lys Asp Gly Cys His Trp Arg Phe Asp Trp Phe Glu Asn Ala Asp
185 190 195

AAC CCT GAC TTC ACC TTT GAG CAG GTT CAG TGC CCC AAG GCT CTC CTC 738
Asn Pro Asp Phe Thr Phe Glu Gln Val Gln Cys Pro Lys Ala Leu Leu
200 205 210

GAC ATC AGT GGA TGC AAG CGT GAT GAC GAC TCC AGC TTC CCT GCC TTC 786
Asp Ile Ser Gly Cys Lys Arg Asp Asp Asp Ser Ser Phe Pro Ala Phe
215 220 225 230

AAG GTT GAT ACC TCG GCC AGC AAG CCC CAG CCC TCC AGC TCC GCT AAG 834
Lys Val Asp Thr Ser Ala Ser Lys Pro Gln Pro Ser Ser Ser Ala Lys
235 240 245

AAG ACC ACC TCC GCT GCT GCT GCC GCT CAG CCC CAG AAG ACC AAG GAT 882
Lys Thr Thr Ser Ala Ala Ala Ala Ala Gln Pro Gln Lys Thr Lys Asp
250 255 260

TCC GCT CCT GTT GTC CAG AAG TCC TCC ACC AAG CCT GCC GCT CAG CCC 930
Ser Ala Pro Val Val Gln Lys Ser Ser Thr Lys Pro Ala Ala Gln Pro
265 270 275

```
GAG CCT ACT AAG CCC GCC GAC AAG CCC CAG ACC GAC AAG CCT GTC GCC        978
Glu Pro Thr Lys Pro Ala Asp Lys Pro Gln Thr Asp Lys Pro Val Ala
    280                 285                 290

ACC AAG CCT GCT GCT ACC AAG CCC GTC CAA CCT GTC AAC AAG CCC AAG       1026
Thr Lys Pro Ala Ala Thr Lys Pro Val Gln Pro Val Asn Lys Pro Lys
295                 300                 305                 310

ACA ACC CAG AAG GTC CGT GGA ACC AAA ACC CGA GGA AGC TGC CCG GCC       1074
Thr Thr Gln Lys Val Arg Gly Thr Lys Thr Arg Gly Ser Cys Pro Ala
                315                 320                 325

AAG ACT GAC GCT ACC GCC AAG GCC TCC GTT GTC CCT GCT TAT TAC CAG       1122
Lys Thr Asp Ala Thr Ala Lys Ala Ser Val Val Pro Ala Tyr Tyr Gln
            330                 335                 340

TGT GGT GGT TCC AAG TCC GCT TAT CCC AAC GGC AAC CTC GCT TGC GCT       1170
Cys Gly Gly Ser Lys Ser Ala Tyr Pro Asn Gly Asn Leu Ala Cys Ala
        345                 350                 355

ACT GGA AGC AAG TGT GTC AAG CAG AAC GAG TAC TAC TCC CAG TGT GTC       1218
Thr Gly Ser Lys Cys Val Lys Gln Asn Glu Tyr Tyr Ser Gln Cys Val
    360                 365                 370

CCC AAC TAAATGGTAG ATCCATCGGT TGTGGAAGAG ACTATGCGTC TCAGAAGGGA        1274
Pro Asn
375

TCCTCTCATG AGCAGGCTTG TCATTGTATA GCATGGCATC CTGGACCAAG TGTTCGACCC     1334

TTGTTGTACA TAGTATATCT TCATTGTATA TATTTAGACA CATAGATAGC CTCTTGTCAG     1394

CGACAACTGG CTACAAAAGA CTTGGCAGGC TTGTTCAATA TTGACACAGT TTCCTCCATA     1454

AAAAAAAAAA AAAAAAAAA                                                  1473
```

INFORMATION FOR SEQ ID NO 4

(i) SEQUENCE CHARACTERISTICS

(A) LENGTH : 376 amino acids
(B) TYPE : amino acid
(D) TOPOLOGY: linear

(ii) MOLECULE TYPE : protein

SEQUENCE DESCRIPTION: SEQ ID NO:4:

```
Met Arg Ser Tyr Thr Leu Leu Ala Leu Ala Gly Pro Leu Ala Val Ser
 1               5                  10                  15

Ala Ala Ser Gly Ser Gly His Ser Thr Arg Tyr Trp Asp Cys Cys Lys
            20                  25                  30

Pro Ser Cys Ser Trp Ser Gly Lys Ala Ala Val Asn Ala Pro Ala Leu
        35                  40                  45

Thr Cys Asp Lys Asn Asp Asn Pro Ile Ser Asn Thr Asn Ala Val Asn
    50                  55                  60

Gly Cys Glu Gly Gly Gly Ser Ala Tyr Ala Cys Thr Asn Tyr Ser Pro
65                  70                  75                  80

Trp Ala Val Asn Asp Glu Leu Ala Tyr Gly Phe Ala Ala Thr Lys Ile
                85                  90                  95

Ser Gly Gly Ser Glu Ala Ser Trp Cys Cys Ala Cys Tyr Ala Leu Thr
            100                 105                 110

Phe Thr Thr Gly Pro Val Lys Gly Lys Lys Met Ile Val Gln Ser Thr
        115                 120                 125

Asn Thr Gly Gly Asp Leu Gly Asp Asn His Phe Asp Leu Met Met Pro
    130                 135                 140

Gly Gly Gly Val Gly Ile Phe Asp Gly Cys Thr Ser Glu Phe Gly Lys
145                 150                 155                 160

Ala Leu Gly Gly Ala Gln Tyr Gly Gly Ile Ser Ser Arg Ser Glu Cys
                165                 170                 175

Asp Ser Tyr Pro Glu Leu Leu Lys Asp Gly Cys His Trp Arg Phe Asp
            180                 185                 190

Trp Phe Glu Asn Ala Asp Asn Pro Asp Phe Thr Phe Glu Gln Val Gln
        195                 200                 205
```

Cys Pro Lys Ala Leu Leu Asp Ile Ser Gly Cys Lys Arg Asp Asp Asp
210 215 220

Ser Ser Phe Pro Ala Phe Lys Val Asp Thr Ser Ala Ser Lys Pro Gln
225 230 235 240

Pro Ser Ser Ser Ala Lys Lys Thr Thr Ser Ala Ala Ala Ala Ala Gln
245 250 255

Pro Gln Lys Thr Lys Asp Ser Ala Pro Val Val Gln Lys Ser Ser Thr
260 265 270

Lys Pro Ala Ala Gln Pro Glu Pro Thr Lys Pro Ala Asp Lys Pro Gln
275 280 285

Thr Asp Lys Pro Val Ala Thr Lys Pro Ala Ala Thr Lys Pro Val Gln
290 295 300

Pro Val Asn Lys Pro Lys Thr Thr Gln Lys Val Arg Gly Thr Lys Thr
305 310 315 320

Arg Gly Ser Cys Pro Ala Lys Thr Asp Ala Thr Ala Lys Ala Ser Val
325 330 335

Val Pro Ala Tyr Tyr Gln Cys Gly Gly Ser Lys Ser Ala Tyr Pro Asn
340 345 350

Gly Asn Leu Ala Cys Ala Thr Gly Ser Lys Cys Val Lys Gln Asn Glu
355 360 365

Tyr Tyr Ser Gln Cys Val Pro Asn
370 375

## Claims

1. A laundry wash solution comprising a solution of a laundry detergent composition comprising conventional detergency ingredients, characterized in that it comprises a cellulase, having a pH optimum of between 5 and 9,5 at a level of from 0.005 to 40 mg/l of said cellulase in said wash solution and a polyvinylpyrrolidone of a molecular weight of from 8 000 to 15.000 at a level of from 5-500 mg/l of said polyvinylpyrrolidone in the wash solution.

2. A laundry wash solution according to claim 1 wherein said cellulase provides at least 10% removal of immobilized radioactive labeled carboxymethylcellulose according to the C14CMC-method at $25x10^{-6}$% by weight of cellulase protein in a laundry test solution.

3. A laundry wash solution according to claim 2 characterized in that the cellulase consists essentially of a homogeneous endoglucanase component which is immunoreactive with a monoclonal antibody raised against a partially purified about 43kD cellulase derived from Humicola insolens, DSM 1800, or which is homologous to said 43kD endoglucanase.

4. A laundry wash solution according to the preceding claims wherein the cellulase is present at a level of from 0.01 mg/l to 10 mg/l of cellulase in the wash solution.

5. A laundry wash solution according to any of the preceding claims wherein the polyvinylpyrrolidone is present at a level of from 15mg/l to 100 mg/l in the wash solution.

6. A laundry wash solution according to any of the preceding claims, wherein said conventional detergency ingredients consists of surfactants and builders.

7. A laundry wash solution according to any of the preceding claims wherein said detergent composition is in a granular form.

8. A laundry wash solution according to claim 7 wherein said detergent composition has a density of from 550 g/l to 950 g/l.

9. A method of washing fabrics, wherein said fabrics are repeatedly washed with a laundry wash solution according to any of the preceding claims.

10. A detergent composition suitable for providing a wash solution according to claims 1-8.

**Patentansprüche**

1. Wäschewaschlösung, umfassend eine Lösung einer Wäschewaschmittelzusammensetzung, die herkömmliche Waschmittelbestandteile umfaßt, **dadurch gekennzeichnet,** daß sie eine Cellulase mit einem pH-Optimum zwischen 5 und 9.5 in einem Anteil von 0.005 bis 40 mg/l der Cellulase in der Waschlösung und ein Polyvinylpyrrolidon mit einem Molekulargewicht von 8.000 bis 15.000 in einem Anteil von 5-500 mg/l des Polyvinylpyrrolidons in der Waschlösung umfaßt.

2. Wäschewaschlösung nach Anspruch 1, wobei die Cellulase eine mindestens 10 %-ige Entfernung immobilisierter, radioaktiv markierter Carboxymethylcellulose gemäß der C14CMC-Methode bei $25X10^{-6}$ Gew.-% Cellulaseprotein in einer Wäschetestlösung ergibt.

3. Wäschewaschlösung nach Anspruch 2, **dadurch gekennzeichnet,** daß die Cellulase im wesentlichen aus einer homogenen Endoglucanasekomponente besteht, welche gegenüber einem monoklonalen Antikörper, der gegenüber einer teilweise gereinigten, etwa 43kD-Cellulase, die von Humicola insolens, DSM 1.800 abgeleitet ist, produziert worden ist, immunoreaktiv oder zu der 43kD Endoglucanase homolog ist.

4. Wäschewaschlösung nach mindestens einem der vorangehenden Ansprüche, wobei die Cellulase in einem Anteil von 0,01 mg/l bis 10 mg/l Cellulase in der Waschlösung vorliegt.

5. Wäschewaschlösung nach mindestens einem der vorangehenden Ansprüche. wobei das Polyvinylpyrrolidon in einem Anteil von 15 mg/l bis 100 mg/l in der Waschlösung vorliegt.

6. Wäschewaschlösung nach mindestens einem der vorangehenden Ansprüche, wobei die herkömmlichen Waschmittelbestandteile aus Tensiden und Buildern bestehen.

7. Wäschewaschlösung nach mindestens einem der vorangehenden Ansprüche. wobei die Waschmittelzusammensetzung in granulärer Form vorliegt.

8. Wäschewaschlösung nach Anspruch 7, wobei die Waschmittelzusammensetzung eine Dichte von 550 g/l bis 950 g/l aufweist.

9. Verfahren zum Waschen von Textilien, wobei die Textilien wiederholt mit einer Wäschewaschlösung gemäß mindestens einem der vorangehenden Ansprüche gewaschen werden.

10. Waschmittelzusammensetzung, welche zum Vorsehen einer Waschlösung gemäß den Ansprüchen 1 - 8 geeignet ist.

**Revendications**

1. Solution de lessive comprenant une composition détergente pour la lessive comprenant des ingrédients de détergence classiques, caractérisée en ce qu'elle comprend une cellulase ayant un pH optimum compris entre 5 et 9,5, en proportion de 0,005 à 40 mg/l de ladite cellulase dans ladite solution de lavage, et une polyvinylpyrrolidone ayant une masse moléculaire de 8 000 à 15 000, en proportion de 5-500 mg/l de ladite polyvinylpyrrolidone dans la solution de lavage.

2. Solution de lessive selon la revendication 1, dans laquelle ladite cellulase fournit au moins 10% d'élimination de carboxyméthylcellulose à marquage radio-isotopique immobilisée, selon la méthode $^{14}$C-CMC à 25x10$^{-6}$% en poids de protéine cellulase dans une solution de lessive test.

3. Solution de lessive selon la revendication 2, caractérisée en ce que la cellulase se compose essentiellement d'un constituant endoglucanase homogène qui est immunoréactif avec un anticorps monoclonal élevé contre une cellulase d'environ 43 kD partiellement purifiée dérivée de Humicola insolens, DSM 1800, ou qui est homologue à ladite endoglucanase de 43 kD.

4. Solution de lessive selon les revendications précédentes, dans laquelle la cellulase est présente en proportion de 0,01 mg/l à 10 mg/l de cellulase dans la solution de lavage.

5. Solution de lessive selon l'une quelconque des revendications précédentes, dans laquelle la polyvinylpyrrolidone est présente en proportion de 15 mg/l à 100 mg/l dans la solution de lavage.

6. Solution de lessive selon l'une quelconque des revendications précédentes. dans laquelle lesdits ingrédients de détergence classiques se composent de tensioactifs et d'adjuvants.

7. Solution de lessive selon l'une quelconque des revendications précédentes, dans laquelle ladite composition détergente est sous forme granulaire.

8. Solution de lessive selon la revendication 7, dans laquelle ladite composition détergente possède une densité de 550 g/l à 950 g/l.

9. Procédé de lavage de tissus, dans lequel lesdits tissus sont lavés de façon répétée avec une solution de lessive selon l'une quelconque des revendications précédentes.

10. Composition détergente utilisable pour fournir une solution de lavage selon les revendications 1-8.